(19) Europäisches Patentamt
European Patent Office
Office européen des brevets

(11) Veröffentlichungsnummer: **0 103 833 B1**

# (12) EUROPÄISCHE PATENTSCHRIFT

(45) Veröffentlichungstag der Patentschrift: **16.08.89**

(51) Int. Cl.⁴: **C 07 D 471/08**, C 07 D 471/10, A 61 K 31/435 // (C07D471/08, 221:00, 221:00)

(21) Anmeldenummer: **83108990.9**

(22) Anmeldetag: **12.09.83**

## (54) Diazabicyclo-(3,3,1)-nonane.

**Teilanmeldung 87108007 eingereicht am 03.06.87.**

(30) Priorität: **18.09.82 DE 3234697**

(43) Veröffentlichungstag der Anmeldung: **28.03.84 Patentblatt 84/13**

(45) Bekanntmachung des Hinweises auf die Patenterteilung: **16.08.89 Patentblatt 89/33**

(84) Benannte Vertragsstaaten: **AT BE CH DE FR GB IT LI LU NL SE**

(56) Entgegenhaltungen:
**EP-A- 0 000 074**
**FR-A- 2 375 235**

(73) Patentinhaber: **Kali-Chemie Pharma GmbH, Hans-Böckler-Allee 20 Postfach 220, D-3000 Hannover 1 (DE)**

(72) Erfinder: **Schön, Uwe, Dr., Dipl.-Chem., Mendelssohnstrasse 49, D-3000 Hannover 1 (DE)**
Erfinder: **Hachmeister, Bernd, Dipl.-Chem., Dr.rer.nat., Mövenkamp 4, D-3004 Isernhagen 1 (DE)**
Erfinder: **Kehrbach, Wolfgang, Dipl.-Chem., Dr.rer.nat., Altenbekener Damm 41, D-3000 Hannover 1 (DE)**
Erfinder: **Kühl, Ulrich, Dr.med.vet., Franzburger Strasse 10, D-3007 Gehrden (DE)**
Erfinder: **Buschmann, Gerd, Dr. med. vet., Matthäikirchstrasse 27, D-3000 Hannover (DE)**

(74) Vertreter: **Lauer, Dieter, Dr., c/o Kali-Chemie AG Postfach 220 Hans-Böckler-Allee 20, D-3000 Hannover 1 (DE)**

**EP 0 103 833 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Diazabicyclo-(3,3,1)-nonane, Verfahren zu ihrer Herstellung, Arzneimittel enthaltend Diazabicyclo-(3,3,1)-nonane und deren pharmazeutisch verwendbare Säureadditionssalze und ein Verfahren zur Herstellung solcher Arzneimittel.

Mehrere Veröffentlichungen über 3,7-Diazabicyclo-(3,3,1)-nonane sprechen für ein großes Interesse in chemischer [z.B. Russian Chem. Rev. 34, 439 (1965); Anm. Ist. Super Sanita 4, 157 (1968); J. Org. Cehm. 33, 355 (1968); Russian Chem. Rev. 42, 190 (1973); Chem. Ber. 110, 3894 ff; Austral. J. Chem. 13 (1966), 129 ff; J. Chem. Soc. 115 (1919), S. 686 ff] und pharmakologischer Hinsicht (z.B. Eur. J. med. Chem. 1977, S. 301–5). Die pharmakologischen Wirkungen reichen von einem geringen lokalanaesthetischen Effekt in J. Chem. Soc. 1951, 1706 über eine Wirkung auf das zentrale Nervensystem in DE-OS 2 658 558 und DE-OS 2 749 584 bis hin zu antiarrhythmischen Eigenschaften in DE-OS 2 428 792, DE-OS 2 726 571 und DE-OS 2 744 248.

Aufgabe der Erfindung ist es, neue Diazabicyclo-(3,3,1)-nonane mit abgeändertem Wirkungsprofil zur Verfügung zu stellen.

Diese Aufgabe wird durch die Erfindung gelöst.

Die Erfindung betrifft neue Verbindungen der allgemeinen Formel I

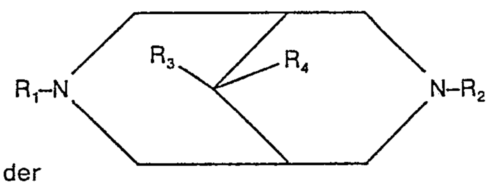

(I)

in der

$R_1$ und $R_2$ unabhängig voneinander Alkyl mit 1 bis 12 C-Atomen, Alkenyl mit 2–12 C-Atomen oder Cycloalkyl mit 3 bis 6 C-Atomen, wobei das Cycloalkyl entweder direkt oder über einen Alkylenrest von 1 bis 3 C-Atomen, vorzugsweise 1 C-Atom mit dem N-Atom verbunden ist, bedeuten und

$R_3$ und $R_4$ entweder unabhängig voneinander Alkyl mit 1 bis 7 C-Atomen bedeuten oder gemeinsam eine Alkylenkette $-(CH_2)_n-$ bilden, mit $n = 3$ bis 6,

mit der Maßgabe, daß

die Reste $R_1$ bis $R_4$ zusammengezählt mindestens 5 Kohlenstoffatome enthalten,

und deren pharmazeutisch verwendbare Säureadditionssalze.

In einer bevorzugten Variante enthalten die Substituenten $R_1$ und/oder $R_2$ jeweils bis 7 Kohlenstoffatome.

Soweit unter $R_1$ und $R_2$ Alkyl verstanden wird, kommt sowohl verzweigtes als auch unverzweigtes Alkyl in Frage. Unverzweigte Alkylreste sind z.B. der Methyl-, Äthyl-, n-Propyl-, n-Butyl-, n-Pentyl-, n-Hexyl- oder n-Heptylrest. Verzweigte Alkylreste sind z.B. der Isopropyl, sek.-Butyl-(=2-Methylpropyl-), 3-Methylbutyl-, 2,2-Dimethylpropyl-, 2-Methylpentyl- und 3,3-Dimethylbutylrest.

Soweit unter $R_1$ und $R_2$ Alkenyl verstanden wird, kommt ebenfalls sowohl verzweigtes als auch unverzweigtes, vorzugsweise unverzweigtes Alkenyl in Frage. Unverzweigte Alkenylreste sind z.B. der Allyl-(=2-Propenyl), 2-Butenyl-, 3-Butenyl-, 2-Pentenyl-, 3-Pentenyl-, 4-Pentenylrest. Ein verzweigter Alkenylrest ist z.B. der 2-Methyl-2-propenylrest.

In einer weiteren Variante können $R_1$ und $R_2$ auch Cycloalkylreste mit 3 bis 6 C-Atomen darstellen. Dabei ist der Cycloalkylrest entweder direkt oder über einen Alkylenrest mit 1–3 C-Atomen, vorzugsweise 1 C-Atom mit dem jeweiligen N-Atom verbunden. Beispiele für solche Reste sind die Cyclopropyl-, Cyclobutyl-, Cyclopentyl-, Cyclohexylgruppe, die, sofern sie über einen Alkylenrest gebunden sind, vorzugsweise über eine Methylengruppe mit dem jeweiligen N-Atom verbunden sind.

Wie bereits angeführt, können $R_1$ und $R_2$ gleiche oder verschiedene Bedeutung besitzen. Bevorzugt wird, daß $R_1$ und $R_2$ gleiche Bedeutung besitzen, insbesondere für den Fall, daß $R_3$ und $R_4$ verschieden sind.

Soweit unter $R_3$ und $R_4$ in einer bevorzugten Variante Alkyl, insbesondere unverzweigtes Alkyl verstanden wird, so gelten die für $R_1$ und $R_2$ gemachten Angaben entsprechend. Bevorzugt enthalten $R_3$ und $R_4$ jeweils 1 bis 7, insbesondere 1 bis 4 Kohlenstoffatome.

$R_3$ und $R_4$ können prinzipiell gleiche oder verschiedene Bedeutung besitzen. Bevorzugt wird, daß $R_3$ und $R_4$ gleiche Bedeutung besitzen, insbesondere für den Fall, daß $R_1$ und $R_2$ verschieden sind.

Ein Sonderfall für $R_3 = R_4$ ist, daß $R_3$ und $R_4$ gemeinsam eine Alkylenkette $-(CH_2)_n-$ bilden. Dabei nimmt n Werte von 3 bis 6, insbesondere 3 bis 5 an.

Ausgewählte Vertreter von Verbindungen der allgemeinen Formel I, worin $R_1$ und $R_2$ verschieden sind, sind 3,7-Diazabicyclo-(3,3,1)-nonane mit folgenden Substituenten:

N-i-Propyl-N'-(2-methylpropyl)-9,9-pentamethylen-,
N-i-Propyl-N'-(cyclohexylmethyl)-9,9-di-n-butyl-,
N-n-Butyl-N'-(2-methylpropyl)-9,9-dimethyl-,
N-n-Butyl-N'-(cyclohexylmethyl)-9,9-dimethyl-,
N-n-Hexyl-N'-methyl-9,9-diäthyl-,
N-(2-Methylpropyl)-N'-(3-butenyl-9,9-di-n-propyl-,
N-n-Butyl-N'-(3-butenyl)-9,9-dimethyl-.

Ausgewählte Vertreter von Verbindungen der allgemeinen Formel I, worin $R_1$ und $R_2$ identisch sind, sind 3,7-Diazabicyclo-(3,3,1)-nonane mit folgenden Substituenten:

N,N'-Diäthyl-9,9-dimethyl-,
N,N'-Di-n-propyl-9,9-diäthyl-,
N,N'-Di-i-propyl-9,9-dimethyl-,
N,N'-Di-i-propyl-9,9-di-n-propyl-,
N,N'-Di-n-butyl-9,9-dimethyl-,
N,N'-9,9-Tetra-n-butyl-,
N,N'-Di-n-butyl-9-methyl-9-äthyl-,
N,N'-Di-(cyclopropyl)-methyl-9,9-tetramethylen-,
N,N'-Di-n-hexyl-9,9-dimethyl-,

N,N'-Di-n-hexyl-9-äthyl-9-n-butyl-,
N,N'-Di-n-hexyl-9,9-tetramethylen-,
N,N'-Di-(cyclohexyl)-methyl-9-methyl-9-äthyl-,
N,N'-Di-(cyclohexyl)-methyl-9,9-pentamethylen-,
N,N'-Di-n-decyl-9,9-dimethyl-,
N,N'-Di-i-propyl-9-methyl-9-n-propyl-,
N,N'-Di-n-butyl-9,9-trimethylen-,
N,N'-Di-(2-propenyl)-9,9-dimethyl-,
N,N'-Di-(3-butenyl)-9,9-pentamethylen-,
N,N'-Di-(3-butenyl)-9,9-dimethyl-,
N,N'-Di-(3-butenyl)-9,9-methyl-9-n-propyl-,

Die Erfindung betrifft weiterhin Arzneimittel enthaltend mindestens eine Verbindung gemäß allgemeiner Formel I oder deren pharmazeutisch verwendbare Säureadditionssalze. Als pharmazeutisch verwendbare Säureadditionssalze eignen sich beispielsweise wasserlösliche und wasserunlösliche Salze von anorganischen oder organischen Säuren wie z.B. das Hydrochlorid, Hydrobromid, Hydrojodid, Phosphat, Nitrat, Sulfat, Perchlorat, Acetat, Citrat, Gluconat, Benzoat, Propionat, Butyrat, Salicylat, Sulfosalicylat, Maleinat, Laurat, Fumarat, Succinat, Oxalat, Tartrat, Stearat, Tosylat (p-Toluolsulfonat), 2-Hydroxy-3-naphthoat, 3-Hydroxy-2-naphthoat, Mesylat (Methansulfonat), Naphthalinsulfonat.

Die Erfindung betrifft weiterhin ein Verfahren zur Herstellung von 3,7-Diazabicyclo-(3,3,1)-nonanen der allgemeinen Formel I und ihrer pharmazeutisch verwendbaren Säureadditionssalze, das dadurch gekennzeichnet ist, daß man

a) entsprechend substituierte Verbindungen der allgemeinen Formel II

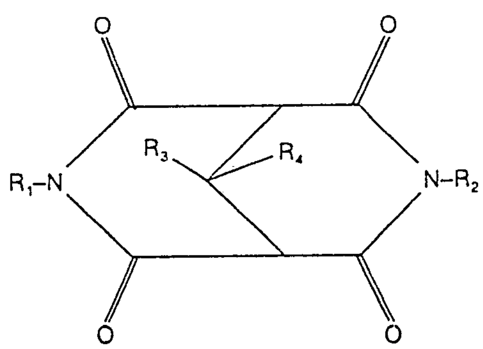 (II)

worin $R_1$, $R_2$, $R_3$ und $R_4$ obige Bedeutung besitzen, reduziert oder

b) Verbindungen der allgemeinen Formel IIIa

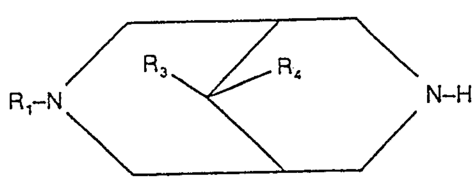 (IIIa)

worin $R_1$, $R_3$ und $R_4$ obige Bedeutung besitzen, mit Verbindungen der allgemeinen Formel $R_2X$, worin $R_2$ obige Bedeutung besitzt und X für eine an sich bekannte Fluchtgruppe steht, umsetzt oder

c) zur Herstellung von Verbindungen der allgemeinen Formel I, worin $R_1$ und $R_2$ identisch sind, Verbindungen der allgemeinen Formel IIIb

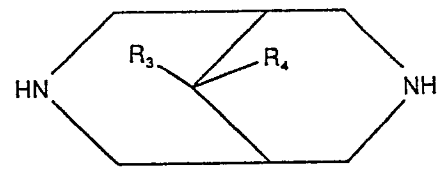 (IIIb)

worin $R_3$ und $R_4$ obige Bedeutung besitzen, mit Verbindungen der allgemeinen Formel $R_1X$, worin $R_1$ und X obige Bedeutung besitzen, umsetzt, und die so erhaltenen Verbindungen der allgemeinen Formel I gewünschtenfalls in ihre pharmazeutisch verwendbaren Säureadditionssalze überführt.

Die Ausgangsverbindungen der allgemeinen Formeln II, IIIa und IIIb können ausgehend von substituierten Dinitrilen der allgemeinen Formel IV

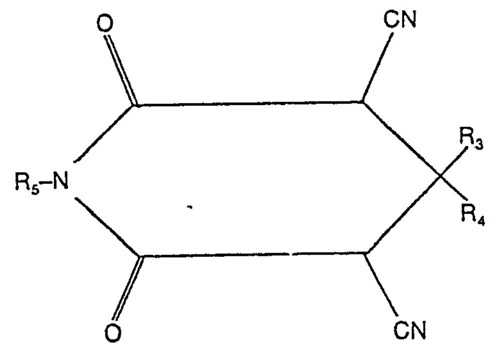 (IV)

oder Mononitrilen der allgemeinen Formel V

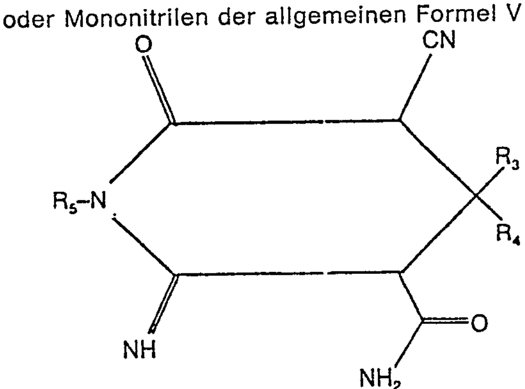 (V)

in denen $R_3$ und $R_4$ obige Bedeutung besitzen und $R_5$ Wasserstoff bedeutet oder die für $R_1$ oben angegebene Bedeutung besitzt, erhalten werden, indem man

a') die Verbindungen der allgemeinen Formeln IV oder V zunächst sauer hydrolysiert zu den Verbindungen der allgemeinen Formel VI

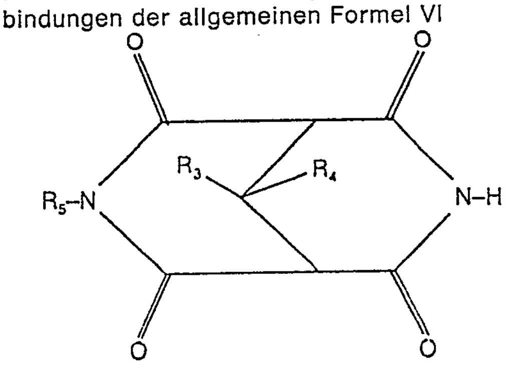 (VI)

worin $R_3$, $R_4$ und $R_5$ obige Bedeutung besitzen, und

b') diese alkyliert und zwar

b'1) für den Fall $R_5$ = H mit $R_2X$, worin X und $R_2$ obige Bedeutung besitzen, monoalkyliert zu einer Verbindung der allgemeinem Formel VIa

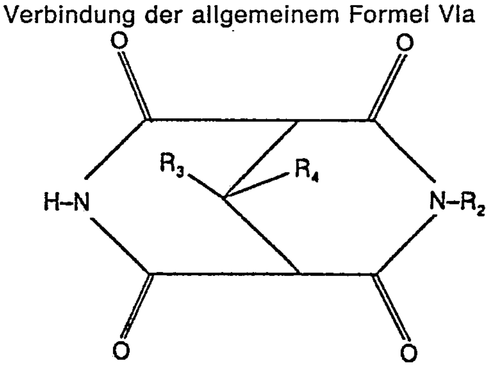
(VIa)

worin $R_2$, $R_3$ und $R_4$ obige Bedeutung besitzen oder

b'2) für den Fall, daß $R_5$ = $R_1$ ist, mit $R_2X$ monoalkyliert zu einer Verbindung der allgemeinen Formel II

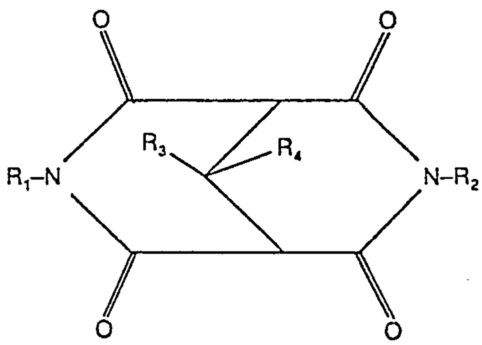
(II)

worin $R_1$, $R_2$, $R_3$ und $R_4$ obige Bedeutung besitzen oder

b'3) für den Fall $R_5$ = H mit $R_1X$ dialkyliert zu einer symmetrisch substituierten Verbindung der allgemeinen Formel IIa

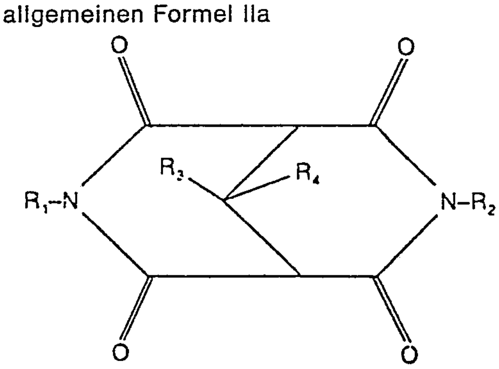
(IIa)

worin $R_1$, $R_3$ und $R_4$ obige Bedeutung besitzen, oder

b'4) für den Fall $R_5$ = H nacheinander mit $R_1X$ und $R_2X$ jeweils monoalkyliert zu einer Verbindung der allgemeinen Formel II, oder

c') zur Herstellung von Verbindungen der allgemeinen Formeln IIIa oder IIIb entsprechend substituierte Verbindungen der allgemeinen Formel VI reduziert zu Verbinduangen der allgemeinen Formeln IIIa oder IIIb.

Die 2,4,6,8-Tetraoxo-3,7-diazabicyclo-(3,3,1)-nonane der allgemeinen Formel VI lassen sich in Analogie zu J. Amer. Chem. Soc. 80, 3915 (1958) darstellen durch Umsetzung der Dinitrile der allgemeinen Formel IV

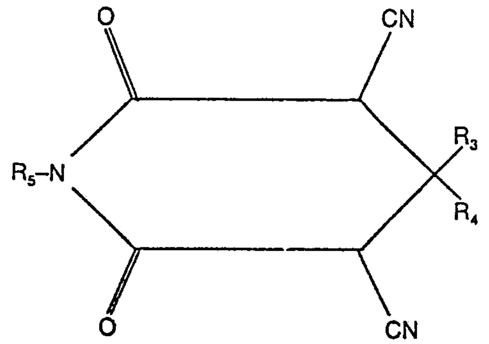
(IV)

oder der Mononitrile der allgemeinen Formel V

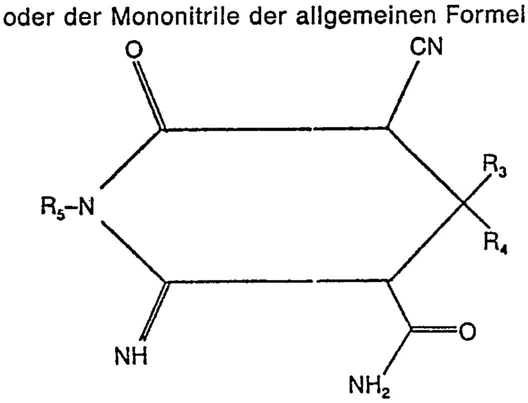
(V)

mit hochprozentigen Säure-Wasser-Gemischen, wobei z.B. als Säuren Schwefelsäure und Phosphorsäure zu nennen sind.

Die Dinitrile der allgemeinen Formel IV sind literaturbekannt oder können in Analogie zu bekannten Verfahren synthetisiert werden, indem man Alkylidenessigester mit Cyanacetamiden kondensiert (Org. Syn. 39, 52) oder Cyanessigester in ammoniakalischer Alkohol-Lösung mit Ketonen zur Reaktion bringt (Org. Syn. 36, 28).

Die Mononitrile der allgemeinen Formel V, welche zum Teil literaturbekannt sind, werden erhalten durch Kondensation des entsprechenden Ketons mit Cyanacetamid im alkalischen Medium [J. Chem. Soc. 99, 422 (1911)]

Bei der Reduktion der 2,4,6,8-Tetraoxo-3,7-diazabicyclo-(3,3,1)-nonane der allgemeinen Formel VI bzw. deren N-Monoalkylprodukte VIa bzw. N,N'Dialkylierungsprodukte II oder IIa kann man auf an sich bekannte Methoden [z.B. J. Amer. Chem. Soc. 78, 2582 (1956) oder Israel. J. Chem. 4, 39 (1966)] zurückgreifen. Als besonders vorteilhaft erweist es sich, als Reduktionsmittel komplexe Metallhydride wie z.B. Lithiumaluminiumhydrid oder Natriumborhydrid in Gegenwart von Lewis-Säuren einzusetzen.

Vorzugsweise verwendet man Lithiumaluminiumhydrid in einem 70 zu 30 Gemisch von Tetrahydrofuran und Toluol. Im Gegensatz zu den oben angeführten Methoden oder zu Eur. J. Med. Chem. 12, 301, (1977) erreicht man in diesem Lösungsmittelgemisch bereits nach relativ kurzen Reaktionszeiten von in der Regel 2 bis 4 Stunden eine vollständige Umsetzung. Ist der Zutritt des Reduktionsmittels aus sterischen Gründen behindert, wie z.B. bei N-i-Propyl-Substitution, so sind längere Reaktionszeiten erforderlich. Es ist vorteilhaft einen Überschuss von 200 bis 400% an Reduktionsmittel einzusetzen.

Sofern Reste $R_1$ und/oder $R_2$ ungesättigten Charakter besitzen, also Alkenyl bedeuten, wird als Reduktionsmittel vorzugsweise Natrium-bis-(2-methoxyethoxy)-dihydroaluminat verwendet.

Die Alkylierung der Tetraoxoverbindungen VI oder VIa oder der durch Reduktion erhaltenen Diazabicyclo(3,3,1)-nonane der allgemeinen Formel IIIa oder IIIb erfolgt unter basischen Bedingungen, beispielsweise mit Natriumhydrid in Dimethylformamid, Alkalicarbonat in Dimethylformamid, Natriummethylat in Methanol, Natriumisopropylat in Isopropanol. Natriumamid in Toluol oder Xylol, nach dem bekannten Prinzip der Phasentransferkatalyse, u.a. Vorzugsweise erfolgt die Alkylierung, insbesondere die der Tetraoxoverbindungen VI, VIa mit Natriumhydrid oder Alkalicarbonat in Dimethylformamid bei erhöhter Temperatur.

Die Ausgangsmaterialien für die beschriebenen Verfahren werden vorzugsweise in stöchiometrischen Mengen eingesetzt. Bei der Alkylierung der Verbindungen VI bzw. IIIa und IIIb verwendet man vorzugsweise einen Überschuss an Deprotonierungs- bzw. Alkylierungsmittel von 25 bis 70%.

Als Alkylierungsmittel werden Verbindungen $R_1X$ bzw. $R_2X$ verwendet, in denen X für eine an sich bekannte Fluchtgruppe steht. Insbesondere werden als Alkylierungsmittel die entsprechenden Alkylhalogenide, -tosylate, -brosylate oder -mesylate, vorzugsweise Alkylhaolgenide, insbesondere -chloride oder -bromide eingesetzt. Es ist auch möglich, das Alkylierungsmittel in situ aus den entsprechenden Alkoholen zu erzeugen, z.B. nach der Methode von Mitsunobu (Synthesis 1981, S. 1), bei der ein separater Zusatz von Deprotonierungsmittel entfällt.

Die Umsetzungen können bei Normaldruck oder auch bei erhöhtem Druck durchgeführt werden, vorzugsweise bei Normaldruck.

Die Umsetzungen erfolgen vorzugsweise in inerten organischen Lösungsmitteln oder Gemischen derselben mit Wasser. Geeignete inerte organische Lösungsmittel sind beispielsweise Äther wie Diäthyläther, Dioxan oder Tetrahydrofuran, Halogenkohlenwasserstoffe wie Methylendichlorid oder Tetrachlorkohlenstoff, Dimethylformamid, Aceton.

Die Reaktionstemperaturen können bei den Verfahren in einem Bereich zwischen 20 und 200 °C variieren, vorzugsweise zwischen 40 und 150 °C.

Die ggf. notwendige Trennung von mono- und dialkylierten Produkten erfolgt in üblicher Weise z.B. durch alkalische Extraktion oder chromatographische Trennung.

Nach den angegebenen Verfahren gelingt es, die unterschiedlichsten Substitutionsmuster zu realisieren. So kann man insbesondere durch Wahl von $R_3$ und $R_4$ in den Ausgangsverbindungen IV bzw. V die symmetrische oder asymmetrische Substitution der 9-Stellungen der resultierenden Diazabicyclo-(3,3,1)-nonane erreichen. Andererseits ist es auch leicht möglich, je nach Wahl der Alkylierungsmittel $R_1X$ und $R_2X$ in VI, VIa, IIIa, oder IIIb N,N'-symmetrisch oder -asymmetrisch substituierte Diazabicyclo-(3,3,1)-nonane zu erhalten.

Für den Fall, daß $R_1$ bzw. $R_5$ und $R_2$ sowie $R_3$ und $R_4$ jeweils verschieden sind, erhält man auf den verschiedenen Stufen ein Gemisch von Stereoisomeren. Die Trennung dieser Stereoisomeren gelingt nach an sich bekannten Methoden wie z.B. fraktionierte Salzfällung, Fraktionierung mittels Säulenchromatographie oder in wäßriger Lösung bei unterschiedlichem pH-Wert. Sind $R_3$ und $R_4$ verschieden, so nimmt man die Trennung vorzugsweise bereits auf der Stufe der Tetraoxoverbindungen VI, VIa, II, oder der durch deren Reduktion erhaltenen Diazabicyclo-(3,3,1)-nonane III vor.

Die erfindungsgemäß erhältlichen Säureadditionssalze erhält man durch an sich bekannte Umsetzung der basischen Verbindungen I mit Säuren, welche pharmazeutisch verwendbare Salze bilden.

Die Verbindungen der allgemeinen Formel I und ihre pharmazeutisch verwendbaren Säureadditionssalze zeichnen sich durch interessante pharmakologische Eigenschaften aus und zeigen insbesondere sauerstoffeinsparende, die Herzfrequenz beeinflussende und herzrhythmisierende Wirkungen. Die neuen Verbindungen zeichnen sich aus durch eine gute Wirksamkeit und hohe physiologische Verträglichkeit. So zeigen die neuen Verbindungen schon in geringen Dosen eine befriedigende antiarrhythmische Wirkung. Darüber hinaus ist eine unerwünschte negative Beeinflussung des Kontraktionsvermögens des Herzens äußerst gering. Das heißt, die Verbindungen weisen ein besonders günstiges Verhältnis von antiarrhythmischen, bzw. die Retraktärzeit des Herzens verlängernder Wirkungen zu negativ inotropen Nebenwirkungen auf und besitzen eine große therapeutische Breite. Bei geringen Dosierungen zeigen sie außerdem überraschenderweise einen positiven inotropen Effekt.

Der Einfluß der Wirksubstanz auf den myokardialen Sauerstoffverbrauch wurde an Tiermodellen untersucht und anhand der Methode von Neill [Neill, W.A., H.H. Levine, R.J. Wagman, R. Gorlin, Circulation Research 12 (1963), 163] berechnet. Die hierzu erforderlichen Kreislaufmeßgrößen systolischer Blutdruck und Herzfrequenz wurden in der Versuchsanordnung nach Buschmann et al. [C. Buschmann, W. Schumacher, R. Budden und U.G. Kühl, J. Cardiovasc. Pharmacol. 2 (1980), 777 bis 795] ermittelt. Wie aus Tabelle 1 ersichtlich, reduziert die Wirksubstanz das Doppelprodukt aus Herzfrequenz und systolischem Blutdruck und führt damit zu einer Sauerstoffeinsparung am Herzen. Dieser Effekt ist sowohl nach intravenöser (i.v.) als auch nach intraduodenaler (i.d.) Gabe vorhanden und ist auf die Wirkung der Testsubstanz zurückzuführen.

Die antiarrhythmische Wirkung der erfindungsgemäßen Verbindungen wurde am intakten Versuchstier anhand experimentell hervorgerufener Rhythmustörungen belegt. Bei einer kontinuierlichen intravenösen Infusion von Aconitin bei Ratten zeichnen sich im Elektrokardiogramm erhebliche Störungen der Herzschlagfolge, wie z.B. ventrikuläre Extrasystolen. Der Einfluß der Wirksubstanz auf mit Aconitin infundierte männliche Wistar-Ratten der Gewichtsklasse 280 bis 350 g gemäß der Methode nach Raschak [M. Raschak, Arzneim. Forsch. (Drug Res) 25 (4) (1975), 639–641] ist in Tabelle 2 aufgeführt. Angegeben ist die Differenz derjenigen Zeit in %, bei der es im Vergleich zu einem Kontrollversuch, bei dem die Wirksubstanz durch das Vehikel (isotonische NaCl-Lösung) ersetzt wurde, nach einer intravenösen Gabe an Wirksubstanz in einer Dosis von 6,0 µmol/kg i.v. (einem zwanzigstel der an der Maus bestimmten i.p. $LD_{50}$; die $LD_{50}$ per os beträgt 1038 mmol/kg) und anschließender Infusion einer pro Zeiteinheit konstanten Aconitin-Dosis zum Auftreten von ventrikulären Extrasystolen (ES) kam.

Tabelle 1

Einfluß auf Herzfrequenz (FRQ), systolischen Blutdruck ($P_s$) und das Doppelprodukt (DP) narkotisierter Ratten

| Testsubstanz der allgemeinen Formel I | Dosis [µmol/kg] | FRQ [1/min] | $P_s$ [mm Hg] | DP [mm Hg/min × 1000] | Änderung DP [Prozent] |
|---|---|---|---|---|---|
| Vorwerte | 0 | 372 | 104 | 39 | – |
| Beispiel3, Nr. 302* | 13.5. i.v. | 174 | 147 | 25 | −36 |
| Vorwerte | 0 | 406 | 101 | 41 | – |
| Beispiel 3, Nr. 302* | 100 i.d. | 238 | 125 | 30 | −27 |

* eingesetzt als Di-Tartrat

Zum Vergleich ist die Wirkung einer äquitoxischen Dosis von Lidocain, einem anerkannten, bereits therapeutisch am Menschen eingesetzten Antiarrhythmikum in Tabelle 2 aufgeführt.

Tabelle 2

Antiarrhythmische Wirkung im Aconitintest an der Ratte

| Testsubstanz | Dosis [µmol/kg] | Zeit bis ES [min] | Änderung [%] |
|---|---|---|---|
| Vehikel | 0 | 6,7 | – |
| Beispiel 3, Nr. 302 eingesetzt als Di-Tartrat | 6,0 | 8,2 | +23 |
| Vehikel | 0 | 6,3 | – |
| Lidocain | 10,0 | 6,6 | + 5 |

Der Nachweis der antiarrhythmischen Wirkung der neuen Wirksubstanz erfolgte außerdem experimentell durch Bestimmung der funktionellen Refraktärzeit des linken Herzvorhofes von weiblichen Albino Pirbright-white Meerschweinchen der Gewichtsklasse 300 bis 400 g mit Hilfe der gepaarten elektrischen Stimulation in Anlehnung an die Methode von Govier [W.C. Govier, J. Pharmacol. Exp. Ther. 148 (1) (1965), 100 bis 105]. Sämtliche zur Zeit in der Therapie bereits eingesetzte Antiarrhythmika unterschiedlicher chemischer Struktur zeichnen sich durch eine Verlängerung der funktonellen Refraktärzeit aus. Zusätzlich gestattet die Methode die Erfassung von Substanzeinwirkungen auf die Kontraktionskraft des Herzmuskels. In der Tabelle 3 ist deshalb als FRP 125% diejenige Konzentration in µmol/l angegeben, bei der es 18 Min. nach Substanzapplikation zu einer Verlängerung der funktionellen Refraktärzeit auf 125% kommt bzw. als F 75% die entsprechende Konzentration, die eine Verminderung der Kontraktionskraft auf 75% des Ausgangswertes bewirkt. Angegeben ist außerdem der Quotient F 75%/FRP 125% aus kontraktionskraftmindernder und refraktärzeitverlängernder Dosis. Dieser Quotient gibt Aufschluß über die therapeutische Breite der antiarrhythmischen Wirkungen am isolierten Organ [K. Greeff, Verhandlung der Deutschen Gesellschaft für Kreislaufforschung 35 (1969), 88 bis 97].

Der direkte Einfluß der Wirksubstanz auf die Herzfrequenz (FRQ) wurde an spontan schlagenden, isolierten rechten Vorhöfen von weiblichen Albino Pirbright-white Meerschweinchen (MS) der Gewichtsklasse von 300 bis 400 g geprüft. In Tabelle 3 ist als FRQ 75% diejenige Konzentration in µmol/l angegeben, bei der es 20 Min. nach der Substanzgabe zu einer Abnahme der Frequenz auf 75% des Ausgangswertes kommt.

Aus der Tabelle 3 ergibt sich, daß die neue Substanz keine nennenswerten unerwünschten negativ inotropen Effekte zeigt, jedoch bereits in sehr niedriger Konzentration eine antiarrhythmische und eine herzfrequenzsenkende Wirkung entfaltet.

Tabelle 3

Einfluß auf die Frequenz (FRQ) spontanschlagender rechter Meerschweinchenvorhöfe sowie auf die Kontraktionskraft (F) und die funktionelle Refraktärperiode (FRP) elektrisch gereizter linker Meerschweinchenvorhöfe

| Testsubstanz. der allgemeinen Formel I | effektive FRQ 75% | Konzentr. F 75% | (µmol/l) FRP 125% | Quotient F 75%/ FRP 125 |
|---|---|---|---|---|
| Beispiel 3, Nr. 302 eingesetzt als Di-Tartrat | 3 | 104 | 1 | 104 |
| Vergleich eingesetzt als Di-Tartrat | etwa 215 | etwa 215 | 122 | etwa 2 |

Tabelle 3 gibt auch Ergebnisse von Vergleichsversuchen an, die mit der bekannten Verbindung N,N'-9,9-Tetramethyldiazabicyclo-(3,3,1)-nonan durchgeführt wurden, also derjenigen Verbindung, die den erfindungsgemäßen Verbindungen strukturell am nächsten steht. Der Vergleich zeigt deutlich die überragende therapeutische Breite der erfindungsgemäßen Verbindungen gegenüber der der bekannten Substanz, obwohl diese ihrerseits, wie gutachtlich aus DE-OS 2 428 792, Tabelle 1 zu entnehmen ist, über der therapeutischen Breite anerkannter, bekannter Verbindungen wie z.B. Lidocain liegt.

Die überlegene Wirkung der erfindungsgemäßen Substanzen ist gekennzeichnet durch die Kombination von sauerstoffeinsparendem rhythmisierendem, die Herzfrequenz beeinflussendem und positiv inotropem Effekt.

Dieses Wirkprofil gestattet einen Einsatz bei der ischämischen Herzkrankheit, bei den lebensbedrohlichen Arrhythmien sowie bei Herzversagen.

Die erfindungsgemäßen Verbindungen zeigen die genannten pharmakologischen Wirkungen im Dosisbereich von 0,1 bis 10 mg/kg. Die Applikation kann dabei enteral oder parenteral erfolgen.

Die Erfindung betrifft auch ein Verfahren zur Herstellung von Arzneimitteln, die mindestens eine Verbindung der allgemeinen Formel I oder deren pharmazeutisch verwendbare Säureadditionssalze enthalten. Dieses Verfahren besteht darin, die genannten Verbindungen mit inerten, pharmazeutisch geeigneten Trägerstoffen zu vermischen und in an sich bekannter Weise in galenische Zubereitungen zu überführen. Solche Zubereitungen können z.B. sein: Tabletten, Dragees, Kapseln, Pulver, Granulate, wäßrige und ölige Suspensionen, Emulsionen, Sirupe oder Lösungen für die orale Anwendung, Suppositorien für die rektale Anwendung oder steril injizierbare Suspensionen oder Lösungen für die parenterale Applikation.

Die nachfolgenden Beispiele sollen die Herstellung der neuen Verbindungen näher erläutern, jedoch den Umfang der Erfindung in keiner Weise beschränken.

Die Beispiele 1 und 2 beschreiben die Herstellung von Zwischenprodukten, Beispiel 3 die Herstellung von Endprodukten.

Beispiel 1

Allgemeine Vorschrift zur Cyclisierung zu den Tetraoxo-3,7-Diazabicyclo-(3,3,1)-nonanen der allgemeinen Formel VI:

20 g Dinitril der allgemeinen Formel IV (hergestellt analog zu Org. Syn. 39, 52) bzw. 20 g Mononitril der allgemeinen Formel V [hergestellt analog zu J. Chem. Soc. 99, 422 (1911)] werden in etwa 100 ml Säure unter Rühren bei 120 °C bis 140 °C bis zur vollständigen Auflösung erhitzt. Nach etwa 10 bis 15 Minuten gießt man den Kolbeninhalt auf Eiswasser. Die ausfallende Tetraoxoverbindung VI wird abgesaugt, gegebenenfalls umkristallisiert (vorzugsweise aus Äthanol) und getrocknet. Nach dieser Arbeitsweise wurden die in Tabelle 4a und 4b angegebenen Verbindungen erhalten.

Tabelle 4a

Herstellung von Tetraoxoverbindungen der allgemeinen Formel mit $R_5$ = H

| Nr. | $R^3$ | $R^4$ | Säure [Vol %] | Schmelzpunkt [°C] |
|---|---|---|---|---|
| 101* | $CH_3$ | $CH_3$ | 60% $H_2SO_4$ | über 350 |
| 102 | $C_2H_5$ | $C_2H_5$ | 60% $H_2SO_4$ | 230 |
| 103 | $n-C_3H_7$ | $n-C_3H_7$ | 60% $H_2SO_4$ | 190 |
| 104 | $n-C_4H_9$ | $n-C_4H_9$ | 85% $H_2SO_4$ | 195–199 |
| 105 | $-(CH_2)_3-$ | | 60% $H_2SO_4$ | über 350 |

Tabelle 4a (Fortsetzung)

Herstellung von Tetraoxoverbindungen der allgemeinen Formel mit $R_5 = H$

| Nr. | $R^3$ | $R^4$ | Säure [Vol %] | Schmelzpunkt [°C] |
|---|---|---|---|---|
| 106* | $-(CH_2)_4$ | | 60% $H_2SO_4$ | über 350 |
| 107* | $-(CH_2)_5-$ | | 60% $H_2SO_4$ | 310 |
| 108* | $CH_3$ | $C_2H_5$ | $H_2SO_4/H_3PO_4$ 1:1 | 324–26 |
| 109* | $CH_3$ | $n-C_3H_7$ | $H_2SO_4/H_3PO_4$ 1:1 | 275 |
| 110 | $C_2H_5$ | $n-C_4H_9$ | 70% $H_2SO_4$ | 140 |

* nicht erfindungsgemäß

Tabelle 4b

Herstellung von Tetraoxoverbindungen der allgemeinen Formel mit $R_5 = R_1$

| Nr. | $R^1$ | $R^3$ | $R^4$ | Säure [Vol %] | Schmelzpunkt [°C] |
|---|---|---|---|---|---|
| 150 | $n-C_4H_9$ | $CH_3$ | $CH_3$ | $H_2SO_4/H_3PO_4$ 1:1 | 175–178 |
| 151 | $-CH_2-CH(CH_3)_2$ | $n-C_3H_7$ | $n-C_3H_7$ | $H_2SO_4/H_3PO_4$ 1:1 | 149–153 |
| 152 | $-CH_2-$ 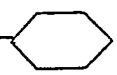 | $n-C_4H_9$ | $n-C_4H_9$ | 60% $H_2SO_4$ | 140 |
| 153 | $n-C_6H_{13}$ | $C_2H_5$ | $C_2H_5$ | $H_2SO_4/H_3PO_4$ 1:1 | Öl |
| 154 | $-CH_2-CH(CH_3)_2$ | $-(CH_2)_5-$ | | $H_2SO_4/H_3PO_4$ 1:1 | 182 |

Beispiel 2

Allgemeine Vorschrift zur Alkylierung der Tetraoxoverbindungen VI

Beispiel 2a:

Dialkylierung der Tetraoxoverbindungen VI ($R_5 = H$) mittels $R_1X$ zu Verbindungen der allgemeinen Formel IIa

0,1 mol der Tetraoxoverbindung VI, die gemäß Beispiel 1 hergestellt wurde, wird in einem ausgeheizten Dreihalskolben eingewogen, mit 200 ml absolutem Dimethylformamid versetzt und auf 60 bis 70 °C erwärmt. Portionsweise gibt man 0,25 mol NaH (berechnet als 100%ig) hinzu und kocht den Ansatz 1 Stunde lang unter Rückfluß. Nach dem Abkühlen der Reaktionslösung tropft man 0,3 mol des in 50 ml absoluten Dimethylformamid gelösten Alkylierungsmittels schnell hinzu und hält den Ansatz dann weitere 3 Stunden am Rückfluß. Der Hauptteil des Lösungsmittels wird im Vakuum abgezogen. Den Rückstand versetzt man mit Methylenchlorid und wäscht mit 20%iger Natriumhydroxidlösung. Die wäßrige Phase wird nochmals mit Methylenchlorid extrahiert. Nach Vereinigen der organischen Phasen werden diese mehrmals mit Wasser gewaschen und über Magnesiumsulfat getrocknet. Der nach Abdestillieren des Lösungsmittels verbleibende Rückstand wird aus Äther/Hexan umkristallisiert.

Alternativ wurde die Alkylierung anstatt mit NaH mit Alkalicarbonat als Base wie folgt durchgeführt:

0,1 mol der Tetraoxoverbindung VI, die gemäß Beispiel 1 hergestellt wurde, wird in einem ausgeheizten Dreihalskolben eingewogen, mit 0,25 mol Alkalicarbonat und 200 ml absolutem Dimethylformamid versetzt und für 1 Stunde auf 120 °C erhitzt. Nach dem Abkühlen der Reaktionslösung tropft man 0,3 mol des in 50 ml abs. Dimethylformamid gelösten Alkylierungsmittels schnell hinzu und hält den Ansatz bis zur vollständigen Umsetzung am Rückfluß. Nach der üblichen Aufarbeitung wird der verbleibende Rückstand aus Äther/Hexan umkristallisiert.

In der Tabelle 5a werden einige der nach diesem Beispiel hergestellten Verbindungen charakterisiert. In der Spalte «Base» bedeuten dabei «A» NaH und «C» Alkalicarbonat.

Beispiel 2b:

Allgemeine Vorschrift zur Monoalkylierung der Tetraoxoverbindung VI ($R_5 = R_1$) mittels $R_2X$ zu Verbindungen der allgemeinen Formel II.

Es wird wie in Beispiel 2a verfahren, wobei nur die jeweils halbe Menge an NaH bzw. Alkalicarbonat sowie Alkylierungsmittel zum Einsatz kommt.

Tabelle 5a

Dialkylierung der Tetraoxoverbindungen VI ($R_5$ = H) mittels $R_1X$ zu Verbindungen der allgemeinen Formel IIa

| Nr. | $R^1$ | $R^2$ | $R^3$ | $R^4$ | X | Base | Schmelzpunkt [°C] |
|---|---|---|---|---|---|---|---|
| 201 | $C_2H_5$ | $C_2H_5$ | $CH_3$ | $Ch_3$ | Br | A | 152–153 |
| 202 | $n-C_4H_9$ | $n-C_4H_9$ | $CH_3$ | $CH_3$ | Br | C | 97 |
| 203 | $n-C_6H_{13}$ | $n-C_6H_{13}$ | $CH_3$ | $CH_3$ | Br | A | 68–69 |
| 204 | $CH_2=CH$<br>$-CH_2-CH_2$ | $CH_2=CH$<br>$-CH_2-CH_2$ | $CH_3$ | $CH_3$ | Br | C | 91 |
| 205 | $i-C_3H_7$ | $i-C_3H_7$ | $CH_3$ | $CH_3$ | Br | A | 99–101 |
| 206 | $n-C_{10}H_{21}$ | $n-C_{10}H_{21}$ | $CH_3$ | $CH_3$ | Br | A | 63 |
| 207 | $n-C_3H_7$ | $n-C_3H_7$ | $C_2H_5$ | $C_2H_5$ | Cl | A | 94 |
| 208 | $i-C_3H_7$ | $i-C_3H_7$ | $n-C_3H_7$ | $n-C_3H_7$ | Br | A | 145–147 |
| 209 | $CH_3$ | $CH_3$ | $n-C_4H_9$ | $n-C_4H_9$ | J | A | 135–137 |
| 210 | $n-C_4H_9$ | $n-C_4H_9$ | $n-C_4H_9$ | $n-C_4H_9$ | Br | A | 73–75 |
| 211 | $n-C_6H_{13}$ | $n-C_6H_{13}$ | $-(CH_2)_4-$ | | Br | A | 70 |
| 212 | $-CH_2-$ 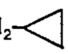 | $-CH_2-$ 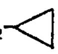 | $-(CH_2)_4-$ | | Cl | A | 160–161 |
| 213 | $-CH_2-$ 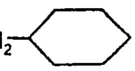 | $-CH_2-$ 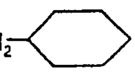 | $-(CH_2)_5-$ | | Br | A | 140 |
| 214 | $CH_2=CH$<br>$-CH_2-CH_2$ | $CH_2=CH$<br>$-CH_2-CH_2$ | $-(CH_2)_5-$ | | Br | C | 120 |
| 215 | $n-C_4H_9$ | $n-C_4H_9$ | $CH_3$ | $C_2H_5$ | Br | A | 82 |
| 216 | $-CH_2-$ 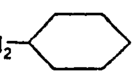 | $-CH_2-$ 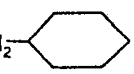 | $CH_3$ | $C_2H_5$ | Br | A | 112–115 |
| 217 | $i-C_3H_7$ | $i-C_3H_7$ | $CH_3$ | $n-C_3H_7$ | Br | A | 103 |
| 218 | $CH_2=CH$<br>$-CH_2-CH_2$ | $CH_2=CH$<br>$-CH_2-CH_2$ | $CH_3$ | $n-C_3H_7$ | Br | A | 57 |
| 219 | $n-C_6H_{13}$ | $n-C_6H_{13}$ | $C_2H_5$ | $n-C_4H_9$ | Br | A | Öl |
| 220 | $CH_2=CH$<br>$-CH_2-CH_2$ | $CH_2=CH$<br>$-CH_2-CH_2$ | $CH_3$ | $CH_3$ | Br | A | 128–130 |
| 221 | $n-C_4H_9$ | $n-C_4H_9$ | $-(CH_2)_3-$ | | Br | A | 110 |

Tabelle 5b

Monoalkylierung der Tetraoxoverbindungen VI ($R_5=R_1$) mittels $R_2X$ zu Verbindungen der allgemeinen Formel II

| Nr. | $R^1$ | $R^2$ | $R^3=R^4$ | X | Base | Schmelzpunkt [°C] |
|---|---|---|---|---|---|---|
| 250 | $n-C_4H_9$ | $-CH_2-CH(CH_3)_2$ | $CH_3$ | Br | A | 80 |
| 251 | $n-C_4H_9$ | $CH_2=CH$<br>$-CH_2-CH_2$ | $CH_3$ | Br | A | 80–83 |
| 252 | $n-C_4H_9$ | $-CH_2-$ 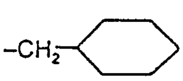 | $CH_3$ | Br | A | 100 |

Tabelle 5b (Fortsetzung)

Monoalkylierung der Tetraoxoverbindungen VI ($R_5 = R_1$) mittels $R_2X$ zu Verbindungen der allgemeinen Formel II

| Nr. | $R^1$ | $R^2$ | $R^3 = R^4$ | X | Base | Schmelzpunkt [°C] |
|---|---|---|---|---|---|---|
| 253 | $CH_3$ | $n-C_6H_{13}$ | $C_2H_5$ | J | A | 93–96 |
| 254 | $i-C_3H_7$ | 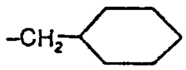 | $n-C_4H_9$ | Br | A | 85 |
| 255 | $-CH_2-CH(CH_3)_2$ | $CH_2=CH$<br>\|<br>$-CH_2-CH_2$ | $n-C_3H_7$ | Br | A | Öl |
| 256 | $i-C_3H_7$ | $-CH_2-CH(CH_3)_2$ | $-(CH_2)_4-$ | Br | A | 101 |

Beispiel 3

Allgemeine Vorschrift zur Reduktion der dialkylierten Tetraoxoverbindungen II oder IIa zu den 3,7-Diazabicyclo-(3,3,1)-nonanen I.

In einem ausgeheizten Dreihalskolben befinden sich 0,1 mol Lithiumaluminiumhydrid in 100 ml einer Lösung aus 70 ml absolutem Tetrahydrofuran und 30 ml absolutem Toluol. Bei 80 °C Ölbadtemperatur tropft man langsam 0,025 mol Tetraoxoverbindung II oder IIa in 100 ml einer 70:30-Mischung von Tetrahydrofuran : Toluol zu.

Man hält den Ansatz 2 bis 4 Stunden lang auf 120 °C und hydrolysiert anschließend unter basischen Bedingungen. Nach Extraktion mit Methylenchlorid und Trocknen der organischen Phase mit Magnesiumsulfat engt man die Lösung ein. Mit Hilfe der Kugelrohrdestillation unter vermindertem Druck werden beispielsweise die in der Tabelle 6a aufgeführten 3,7-Diazabicyclo-(3,3,1)-nonane erhalten.

N,N'-Disubstituierte Tetraoxoverbindungen II oder IIa mit N-Alkenylsubstituenten wurden analog mit Natrium-bis-(2-methoxyethoxy)-dihydroaluminat (Handelsname Red-Al® in Toluol als Reduktionssystem reduziert. Die Reaktionsprodukte sind in Tabelle 6b angeführt.

Die folgenden Beispiele 4 bis 6 beschreiben pharmazeutische Zubereitungen, enthaltend die erfindungsgemäßen Wirkstoffe sowie die Herstellung solcher pharmazeutischer Zubereitungen.

Tabelle 6a:

Reduktion der Tetraoxoverbindungen II bzw IIa zu den 3,7-Diazabicyclo-(3,3,1)-nonanen der allgemeinen Formel I

| Nr. | $R^1$ | $R^2$ | $R^3$ | $R^4$ | Siedepunkt (°C bei 0,1 Torr) |
|---|---|---|---|---|---|
| 301 | $C_2H_5$ | $C_2H_5$ | $CH_3$ | $CH_3$ | 160–170 |
| 302 | $n-C_4H_9$ | $n-C_4H_9$ | $CH_3$ | $CH_3$ | 130 |
| 303 | $n-C_6H_{13}$ | $n-C_6H_{13}$ | $CH_3$ | $CH_3$ | 210–220 |
| 304 | $i-C_3H_7$ | $i-C_3H_7$ | $CH_3$ | $CH_3$ | 100–120 |
| 305 | $n-C_{10}H_{21}$ | $n-C_{10}H_{21}$ | $CH_3$ | $CH_3$ | 230 |
| 306 | $n-C_3H_7$ | $n-C_3H_7$ | $C_2H_5$ | $C_2H_5$ | 140–150 |
| 307 | $i-C_3H_7$ | $i-C_3H_7$ | $n-C_3H_7$ | $n-C_3H_7$ | 150–160 |
| 308 | $n-C_6H_{13}$ | $n-C_6H_{13}$ | $-(CH_2)_4-$ | | 250 |
| 309 | 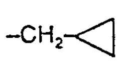 | 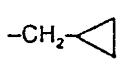 | | $-(CH_2)_4-$ | 230 |
| 310 | $n-C_4H_9$ | $n-C_4H_9$ | $CH_3$ | $C_2H_5$ | 180–200 |
| 311 | $n-C_6H_{13}$ | $n-C_6H_{13}$ | $C_2H_5$ | $n-C_4H_9$ | 230 |
| 312 | $n-C_6H_{13}$ | $CH_3$ | $C_2H_5$ | $C_2H_5$ | 130 |
| 313 | 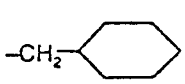 | 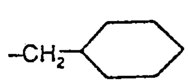 | | $-(CH_2)_5-$ | 102* |
| 314 | 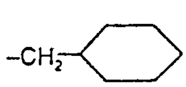 | 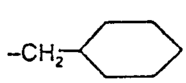 | $CH_3$ | $C_2H_5$ | 36* |

* = Schmelzpunkt

Tabelle 6a: Fortsetzung

| Nr. | $R^1$ | $R^2$ | $R^3$ | $R^4$ | Siedepunkt (°C bei 0,1 Torr) |
|---|---|---|---|---|---|
| 315 | $i-C_3H_7$ | $i-C_3H_7$ | $CH_3$ | $n-C_3H_7$ | 150 |
| 316 | $i-C_3H_7$ | $-CH_2-CH(CH_3)_2$ | | $-(CH_2)_5-$ | 150 |
| 317 | $i-C_3H_7$ | 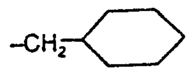 | $n-C_4H_9$ | $n-C_4H_9$ | 190–200 |
| 318 | $n-C_4H_9$ | 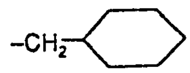 | $CH_3$ | $CH_3$ | 160 |
| 319 | $n-C_4H_9$ | $-CH_2-CH(CH_3)_2$ | $CH_3$ | $CH_3$ | 160–170 |
| 320 | $n-C_4H_9$ | $n-C_4H_9$ | | $-(CH_2)_3-$ | 170 |
| 321 | $n-C_4H_9$ | $n-C_4H_9$ | $n-C_4H_9$ | $n-C_4H_9$ | 210 |

Tabelle 6b:

Reduktion von N-Alkenyl-substituierten Tetraoxoverbindungen II bzw. IIa zu den 3,7-Diazabicyclo-(3,3,1)-nonanen der allgemeinen Formel I

<table>
<tr><th>Nr.</th><th>$R^1$</th><th>$R^2$</th><th>$R^3$</th><th>$R^4$</th><th>Siedepunkt (°C bei 0,1 Torr)</th></tr>
<tr><td>350</td><td>$-CH_2-CH=CH_2$</td><td>$-CH_2-CH=CH_2$</td><td>$CH_3$</td><td>$CH_3$</td><td>160</td></tr>
<tr><td>351</td><td>$CH_2=CH$<br>|<br>$-CH_2-CH_2$</td><td>$CH_2=CH$<br>|<br>$-CH_2-CH_2$</td><td></td><td>$-(CH_2)_5-$</td><td>175</td></tr>
<tr><td>352</td><td>$CH_2=CH$<br>|<br>$-CH_2-CH_2$</td><td>$CH_2=CH$<br>|<br>$-CH_2-CH_2$</td><td>$CH_3$</td><td>$CH_3$</td><td>150</td></tr>
<tr><td>353</td><td>$-CH_2-CH(CH_3)_2$</td><td>$CH_2=CH_2$<br>|<br>$-CH_2-CH$</td><td>$n-C_3H_7$</td><td>$n-C_3H_7$</td><td>170</td></tr>
<tr><td>354</td><td>$n-C_4H_9$</td><td>$CH_2=CH_2$<br>|<br>$-CH_2-CH$</td><td>$CH_3$</td><td>$CH_3$</td><td>165</td></tr>
<tr><td>355</td><td>$CH_2=CH$<br>|<br>$-CH_2-CH_2$</td><td>$CH_2=CH$<br>|<br>$-CH_2-CH_2$</td><td>$CH_3$</td><td>$n-C_3H_7$</td><td>130</td></tr>
</table>

Beispiel 4 – Tabletten

Zusammensetzung:

| | |
|---|---|
| Wirkstoff (Beispiel 3, Nr. 302, Ditartrat) | 20 Teile |
| Maisstärke | 30 Teile |
| Lactose | 55 Teile |
| Kollidon 25" | 5 Teile |
| Magnesiumstearat | 2 Teile |
| Hydriertes Rizinusöl | 1 Teil |
| Total | 113 Teile |

Herstellungsvorschrift:

Der Wirkstoff wird mit Maisstärke und feinpulvriger Lactose in einem Mischer vermischt. Die entstandene Mischung wird mit einer 20%igen Lösung von Polyvinylpyolidon, (Kollidon 25, Fa. BASF) in Isopropanol durchfeuchtet. Falls erforderlich, wird weiteres Isopropanol hinzugefügt. Das feuchte Granulat wird durch ein 2 mm-Sieb passiert, bei 40 °C auf Horden getrocknet und anschließend durch ein 1 mm-Sieb (Frewitt-Maschine) passiert. Nach dem Vermischen des Granulats mit Magnesiumstearat und hydriertem Rizinusöl

werden damit Tabletten von 113 mg gepreßt, so daß jede Tablette 20 mg Wirkstoff enthält.

Beispiel 5 – Kapseln

Zusammensetzung:

| | |
|---|---|
| Wirkstoff (Beispiel 3, Nr. 302, Ditartrat) | 20 Teile |
| Maisstärke | 20 Teile |
| Lactose | 45 Teile |
| Kollidon 25® | 3 Teile |
| Magnesiumstearat | 1,5 Teile |
| Aerosil 200® | 0,5 Teile |
| Total | 90 Teile |

Herstellungsvorschrift:

Der Wirkstoff wird mit Maisstärke und feinpulvriger Lactose in einem Mischer vermischt. Die entstandene Mischung wird mit einer 20%igen Lösung von Polyvinylpyrrolidon (Kollidon 25) in Isopropanol durchgefeuchtet. Falls erforderlich, wird weiteres Isopropanol hinzugefügt. Das feuchte Granulat wird durch ein 1,6 mm-Sieb (Frewitt) passiert, bis 40 °C auf Horden getrocknet und anschließend durch ein 1 mm-Sieb (Frewitt) passiert. Nach dem Vermischen des Granulats mit Magnesiumstearat und Kieselsäureaerogel (Aerosil 200, Fa. Degussa) füllt man davon jeweils 90 mg mittels automatischer Kapselmaschine in Hartgelatinekapseln der Größe 4 ab, so daß jede Kapsel 20 mg Wirkstoff enthält.

Beispiel 6 – Ampullen

Zusammensetzung (pro Ampulle):

| | |
|---|---|
| Wirkstoff Beispiel 3, Nr. 302, Ditartrat) | 5 mg |
| Natriumchlorid | 16 mg |
| Wasser für Injektionszwecke | ad 2,0 ml |

Herstellungsvorschrift:

Man löst Natriumchlorid in Wasser für Injektionszwecke auf, fügt den Wirkstoff hinzu und löst unter Rühren. Mit genügend Wasser für Injektionszwecke wird bis zum Endvolumen aufgefüllt. Der Ansatz wird durch ein Membranfilter 0,25 μ passiert. Man füllt in Braunglasampullen jeweils 2,15 ml ab und schmilzt sie zu. Bei 121 °C wird 30 Minuten lang mit Dampf sterilisiert. 2 ml Injektionslösung enthalten 5 mg Wirkstoff.

**Patentansprüche für die Vertragsstaaten BE, CH, DE, FR, GB, IT, LI, LU, NL, SE**

1. Verbindungen der allgemeinen Formel I

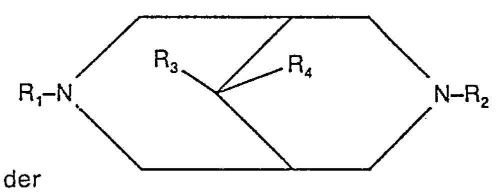

(I)

in der

$R_1$ und $R_2$ unabhängig voneinander Alkyl mit 1 bis 12 C-Atomen, Alkenyl mit 2 bis 12 C-Atomen oder Cycloalkyl mit 3 bis 6 C-Atomen, wobei das Cycloalkyl entweder direkt oder über einen Alkylenrest von 1 bis 3 C-Atomen, vorzugsweise 1 C-Atom mit dem N-Atom verbunden ist, bedeuten und

$R_3$ und $R_4$ entweder unabhängig voneinander Alkyl mit 1 bis 7 C-Atomen bedeuten oder gemeinsam eine Alkylenkette $-(CH_2)_n-$ bilden, mit n = 3 bis 6,

mit der Maßgabe, daß

die Reste $R_1$ bis $R_4$ zusammengezählt mindestens 5 Kohlenstoffatome enthalten,

und deren pharmazeutisch verwendbare Säureadditionssalze.

2. Verbindungen nach Anspruch 1, in denen $R_1$ und/oder $R_2$ jeweils eine Alkyl- oder Cycloalkylalkylgruppe mit bis zu 7 Kohlenstoffatomen oder eine Cycloalkylgruppe mit bis zu 6 Kohlenstoffatomen enthalten.

3. Verbindungen nach einem der Ansprüche 1 oder 2, in denen $R_1$ und/oder $R_2$ jeweils für unverzweigtes Alkyl oder Alkenyl stehen.

4. Verbindungen nach einem der Ansprüche 1 oder 2, in denen $R_1$ und/oder $R_2$ jeweils für verzweigtes Alkyl oder Alkenyl stehen.

5. Verbindungen nach einem der Ansprüche 1 bis 4, in denen die Reste $R_1$ und $R_2$ gleiche Bedeutung besitzen.

6. Verbindungen nach einem der vorhergehenden Ansprüche, in denen $R_3$ und $R_4$ jeweils für Alkyl mit bis zu 4 Kohlenstoffatomen stehen.

7. Verbindungen nach einem der vorhergehenden Ansprüche, in denen $R_3$ und/oder $R_4$ jeweils für unverzweigtes Alkyl stehen.

8. Verbindungen nach einem der vorhergehenden Ansprüche, in denen $R_3$ und $R_4$ gleiche Bedeutung besitzen.

9. Verbindungen nach einem der Ansprüche 1 bis 5, in denen $R_3$ und $R_4$ gemeinsam eine Alkylenkette $-(CH_2)_n-$ bilden mit n = 3 bis 5.

10. Arzneimittel enthaltend mindestens eine Verbindung gemäß Anspruch 1 oder deren pharmazeutisch verwendbare Säureadditionssalze.

11. Verfahren zur Herstellung von Verbindungen der allgemeinen Formel I, worin $R_1$, $R_2$, $R_3$ und $R_4$ die in Anspruch 1 angegebene Bedeutung besitzen, und deren pharmazeutisch verwendbaren Säureadditionssalzen, dadurch gekennzeichnet, daß man

a) entsprechend substituierte Verbindungen der allgemeinen Formel II

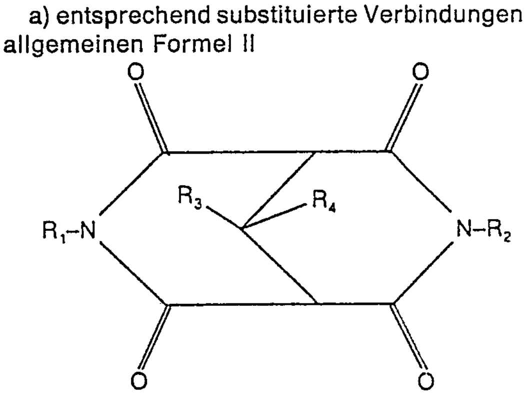

(II)

worin $R_1$, $R_2$, $R_3$ und $R_4$ obige Bedeutung besitzen, reduziert oder

b) Verbindungen der allgemeinen Formel IIIa

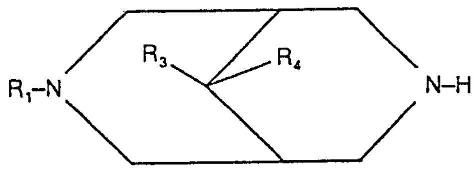
(IIIa)

worin $R_1$, $R_3$ und $R_4$ obige Bedeutung besitzen, mit Verbindungen der Formel $R_2X$, worin $R_2$ obige Bedeutung besitzt und X für eine an sich bekannte Fluchtgruppe steht, umsetzt oder

c) zur Herstellung von Verbindungen der allgemeinen Formel I, worin $R_1$ und $R_2$ identisch sind, Verbindungen der allgemeinen Formel IIIb

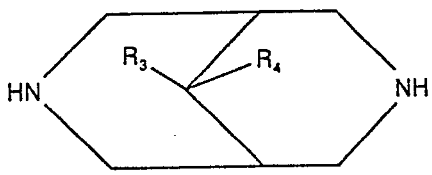
(IIIb)

worin $R_3$ und $R_4$ obige Bedeutung besitzen, mit Verbindungen der allgemeinen Formel $R_1X$, worin $R_1$ und X obige Bedeutung besitzen, umsetzt, und die so erhaltenen Verbindungen der allgemeinen Formel I gewünschtenfalls in ihre pharmazeutisch verwendbaren Säureadditionssalze überführt.

**Patentansprüche für den Vertragsstaat AT**

1. Verfahren zur Herstellung von Verbindungen der allgemeinen Formel I

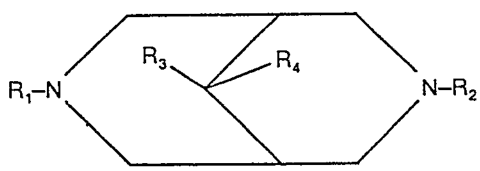
(I)

in der

$R_1$ und $R_2$ unabhängig voneinander Alkyl mit 1 bis 12 C-Atomen, Alkenyl mit 2 bis 12 C-Atomen oder Cycloalkyl mit 3 bis 6 C-Atomen, wobei das Cycloalkyl entweder direkt oder über einen Alkylenrest von 1 bis 3 C-Atomen, vorzugsweise 1 C-Atom mit dem N-Atom verbunden ist, bedeuten und

$R_3$ und $R_4$ entweder unabhängig voneinander Alkyl mit 1 bis 7 C-Atomen bedeuten oder gemeinsam eine Alkylenkette $-(CH_2)_n-$ bilden, mit n = 3 bis 6,

mit der Maßgabe, daß

die Reste $R_1$ bis $R_4$ zusammengezählt mindestens 5 Kohlenstoffatome enthalten,

und deren pharmazeutisch verwendbaren Säureadditionssalzen, dadurch gekennzeichnet, daß man

a) entsprechend substituierte Verbindungen der allgemeinen Formel II

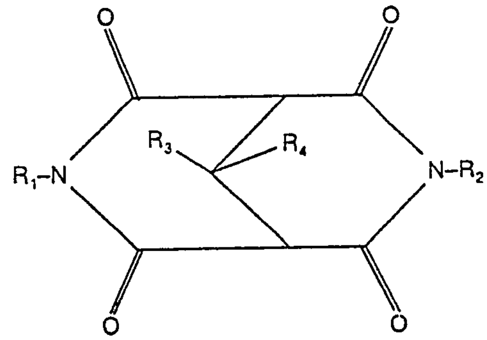
(II)

worin $R_1$, $R_2$, $R_3$ und $R_4$ obige Bedeutung besitzen, reduziert oder

b) Verbindungen der allgemeinen Formel IIIa

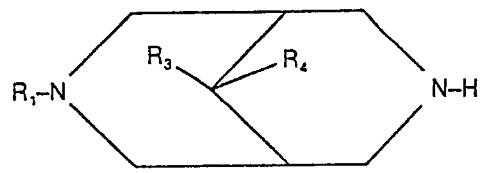
(IIIa)

worin $R_1$, $R_3$ und $R_4$ obige Bedeutung besitzen, mit Verbindungen der allgemeinen Formel $R_2X$, worin $R_2$ obige Bedeutung besitzt und X für eine an sich bekannte Fluchtgruppe steht, umsetzt oder

c) zur Herstellung von Verbindungen der allgemeinen Formel I, worin $R_1$ und $R_2$ identisch sind, Verbindungen der allgemeinen Formel IIIb

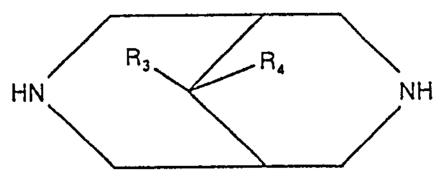
(IIIb)

worin $R_3$ und $R_4$ obige Bedeutung besitzen, mit Verbindungen der allgemeinen Formel $R_1X$, worin $R_1$ und X obige Bedeutung besitzen, umsetzt, und die so erhaltenen Verbindungen der allgemeinen Formel I gewünschtenfalls in ihre pharmazeutischen verwendbaren Säureadditionssalze überführt.

2. Verfahren nach Anspruch 1, dadurch gekennzeichnet, daß Verbindungen der allgemeinen Formel I hergestellt werden, in denen $R_1$ und/oder $R_2$ jeweils eine Alkyl- oder Cycloalkylalkylgruppe mit bis zu 7 Kohlenstoffatomen oder eine Cycloalkylgruppe mit bis zu 6 Kohlenstoffatomen enthalten.

3. Verfahren nach einem der Ansprüche 1 oder 2, dadurch gekennzeichnet, daß Verbindungen der allgemeinen Formel I hergestellt werden, in denen $R_1$ und/oder $R_2$ jeweils für unverzweigtes Alkyl oder Alkenyl stehen.

4. Verfahren nach einem der Ansprüche 1 oder 2, dadurch gekennzeichnet, daß Verbindungen der allgemeinen Formel I hergestellt werden, in denen $R_1$ und/oder $R_2$ jeweils für verzweigtes Alkyl oder Alkenyl stehen.

5. Verfahren nach einem der Ansprüche 1 bis 4, dadurch gekennzeichnet, daß Verbindungen der allgemeinen Formel I hergestellt werden, in denen die Reste $R_1$ und $R_2$ gleiche Bedeutung besitzen.

6. Verfahren nach einem der vorhergehenden Ansprüche, dadurch gekennzeichnet, daß Verbindungen der allgemeinen Formel I hergestellt werden, in denen $R_3$ und $R_4$ jeweils für Alkyl mit bis zu 4 Kohlenstoffatomen stehen.

7. Verfahren nach einem der vorhergehenden Ansprüche, dadurch gekennzeichnet, daß Verbindungen der allgemeinen Formel I hergestellt werden, in denen $R_3$ und/oder $R_4$ jeweils für unverzweigtes Alkyl stehen.

8. Verfahren nach einem der vorhergehenden Ansprüche, dadurch gekennzeichnet, daß Verbindungen der allgemeinen Formel I hergestellt werden, in denen $R_3$ und $R_4$ gleiche Bedeutung besitzen.

9. Verfahren nach einem der Ansprüche 1 bis 5, dadurch gekennzeichnet, daß Verbindungen der allgemeinen Formel I hergestellt werden, in denen $R_3$ und $R_4$ gemeinsam eine Alkylenkette $-(CH_2)_n-$ bilden mit n = 3 bis 5.

**Claims for the contracting countries BE, CH, DE, FR, GB, IT, LI, LU, NL, SE**

1. Compounds of the general Formula I,

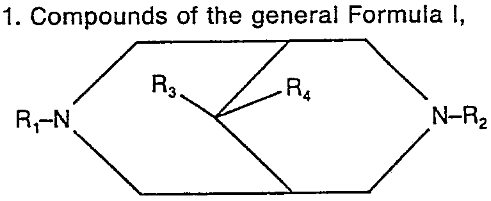
(I)

wherein

$R_1$ and $R_2$, independent of each other, are alkyl with 1 to 12 C atoms, alkenyl with 2 to 12 C atoms or cycloalkyl with 3 to 6 C atoms, the cycloalkyl being connected either directly or via an alkylene radical of 1 to 3 C atoms, preferably 1 C atom, with the N atom, and

$R_3$ and $R_4$ are either alkyl with 1 to 7 C atoms, independent of each other, or together form an alkylene chain $-(CH_2)_n-$ with n = 3 to 6,

with the proviso that

the radicals $R_1$ to $R_4$ added together contain at least 5 carbon atoms,

and their pharmaceutically usable acid addition salts.

2. Compounds according to Claim 1, in which $R_1$ and/or $R_2$ each contain an alkyl or cycloalkyl group with up to 7 carbon atoms or a cycloalkyl group with up to 6 carbon atoms.

3. Compounds according to one of Claims 1 or 2, in which $R_1$ and/or $R_2$ each stand for un-branched alkyl or alkenyl.

4. Compounds according to one of Claims 1 or 2, in which $R_1$ and/or $R_2$ each stand for branched alkyl or alkenyl.

5. Compounds according to one of Claims 1 to 4, in which the radicals $R_1$ and $R_2$ have the same meaning.

6. Compounds according to one of the preceding Claims, in which $R_3$ and $R_4$ each stand for alkyl with up to 4 carbon atoms.

7. Compounds according to one of the preceding Claims, in which $R_3$ and/or $R_4$ each stand for un-branched alkyl.

8. Compounds according to one of the preceding Claims, in which $R_3$ and $R_4$ have the same meaning.

9. Compounds according to one of Claims 1 to 5, in which $R_3$ and $R_4$ together form an alkylene chain $-(CH_2)_n-$, with n = 3 to 5.

10. Medicament containing at least one compound according to Claim 1 or its pharmaceutically usable acid addition salts.

11. A method for producing compounds of general Formula I, wherein $R_1$, $R_2$, $R_3$ and $R_4$ have the meanings given in Claim 1, and their pharmaceutically usable acid addition salts, characterised in that

a) correspondingly substituted compounds of the general Formula II,

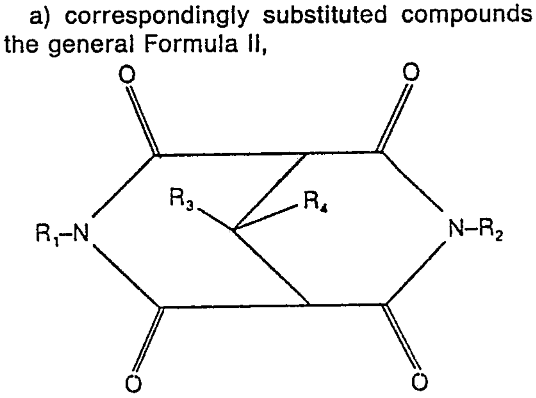
(II)

wherein $R_1$, $R_2$, $R_3$ and $R_4$ have the above meanings, are reduced, or

b) compounds of the general Formula IIIa,

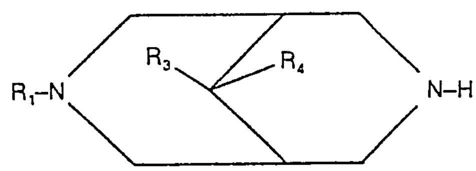
(IIIa)

wherein $R_1$, $R_3$ and $R_4$ have the above meanings, are reacted with compounds of the formula $R_2X$, wherein $R_2$ has the above meaning and X stands for a known easily-cleavable group, or

c) to produce compounds of general Formula I, wherein $R_1$ and $R_2$ are identical, compounds of the general Formula IIIb,

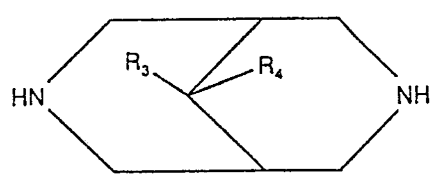
(IIIb)

wherein $R_3$ and $R_4$ have the above meanings, are reacted with compounds of the general formula $R_1X$, wherein $R_1$ and X have the above meanings, and the resulting compounds of general Formula I are converted into their pharmaceutically usable acid addition salts if desired.

**Claims for the contracting country AT**

1. A method for producing compounds of the general Formula I,

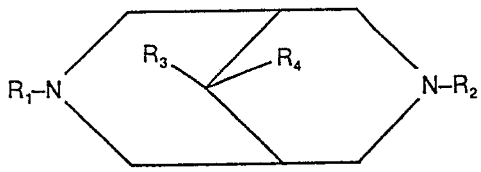
(I)

wherein

$R_1$ and $R_2$, independent of each other, are alkyl with 1 to 12 C atoms, alkenyl with 2 to 12 C atoms or cycloalkyl with 3 to 6 C atoms, the cycloalkyl being connected either directly or via an alkylene radical of 1 to 3 C atoms, preferably 1 C atom, with the N atom, and

$R_3$ and $R_4$ are either alkyl with 1 to 7 C atoms, independent of each other, or together form an alkylene chain $-(CH_2)_n-$ with n = 3 to 6,

with the proviso that

the radicals $R_1$ to $R_4$ added together contain at least 5 carbon atoms,

and their pharmaceutically usable acid addition salts, characterised in that

a) correspondingly substituted compounds of the general Formula II,

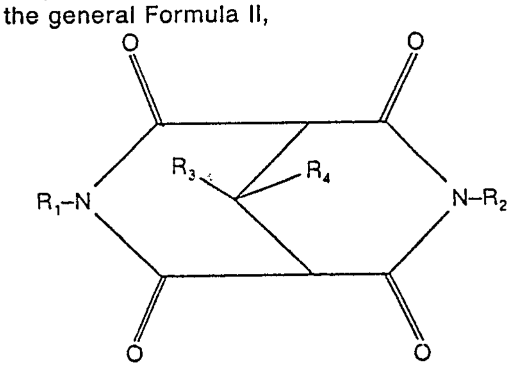
(II)

wherein $R_1$, $R_2$, $R_3$ and $R_4$ have the above meanings, are reduced, or

b) compounds of the general Formula IIIa,

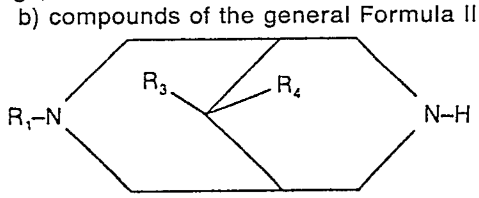
(IIIa)

wherein $R_1$, $R_3$ and $R_4$ have the above meanings, are reacted with compounds of the general formula $R_2X$, wherein $R_2$ has the above meaning and X stands for a known easily-cleavable group, or

c) to produce compounds of general Formula I, wherein $R_1$ and $R_2$ are identical, compounds of the general Formula IIIb,

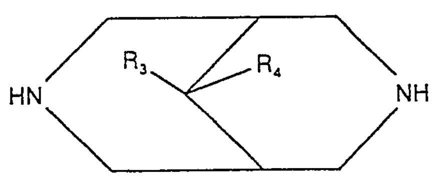
(IIIb)

wherein $R_3$ and $R_4$ have the above meanings, are reacted with compounds of the general formula $R_1X$, wherein $R_1$ and X have the above meanings, and the resulting compounds of general Formula I are converted into their pharmaceutically usable acid addition salts if desired.

2. A method according to Claim 1, characterised in that compounds of general Formula I are produced, in which $R_1$ and/or $R_2$ each contain an alkyl or cycloalkylalkyl group with up to 7 carbon atoms or a cycloalkyl group with up to 6 carbon atoms.

3. A method according to one of Claims 1 or 2, characterised in that compounds of general Formula I are produced, in which $R_1$ and/or $R_2$ each stand for unbranched alkyl or alkenyl.

4. A method according to one of Claims 1 or 2, characterised in that compounds of general Formula I are produced, in which $R_1$ and/or $R_2$ each stand for branched alkyl or alkenyl.

5. A method according to one of Claims 1 to 4, characterised in that compounds of general Formula I are produced, in which the radicals $R_1$ and $R_2$ have the same meaning.

6. A method according to one of the preceding Claims, characterised in that compounds of general Formula I are produced, in which $R_3$ and $R_4$ each stand for alkyl with up to 4 carbon atoms.

7. A method according to one of the preceding Claims, characterised in that compounds of general Formula I are produced, in which $R_3$ and/or $R_4$ each stand for un-branched alkyl.

8. A method according to one of the preceding Claims, characterised in that compounds of general Formula I are produced, in which $R_3$ and $R_4$ have the same meaning.

9. A method according to one of Claims 1 to 5, characterised in that compounds of general Formula I are produced, in which $R_3$ and $R_4$ together form an alkylene chain $-(CH_2)_n-$, with n = 3 to 5.

**Revendications pour les états contractants BE, CH, DE, FR, GB, IT, LI, LU, NL, SE**

1. Composés de formule générale I

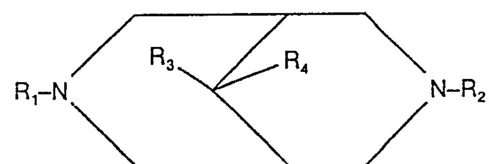
(I)

dans laquelle

$R_1$ et $R_2$, indépendamment l'un de l'autre, sont chacun un radical alkyle ayant de 1 à 12 atomes de carbone, alcényle ayant de 2 à 12 atomes de carbone ou cycloalkyle ayant de 3 à 6 atomes de carbone, le radical cycloalkyle étant lié à l'atome N directement ou par l'intermédiaire d'un radical alkylène ayant de 1 à 3 atomes de carbone, de préférence 1 atome de carbone, et

$R_3$ et $R_4$, indépendamment l'un de l'autre, sont chacun un radical alkyle ayant de 1 à 7 atomes de carbone, ou forment ensemble un enchaînement alkylène $-(CH_2)_n-$, avec n = 3 à 6,

à la condition que les radicaux $R_1$ à $R_4$ contiennent au total au moins 5 atomes de carbone,

et leurs sels d'addition avec un acide, pharmaceutiquement utilisables.

2. Composés selon la revendication 1, dans lesquels chacun des radicaux $R_1$ et/ou $R_2$ contient un groupe alkyle ou cycloalkylalkyle ayant jusqu'à

7 atomes de carbone, ou un groupe cycloalkyle ayant jusqu'à 6 atomes de carbone.

3. Composés selon l'une des revendications 1 ou 2, dans lesquels chacun des radicaux $R_1$ et/ou $R_2$ représente un radical alkyle ou alcényle non-ramifié.

4. Composés selon l'une des revendications 1 ou 2, dans lesquels chacun des radicaux $R_1$ et/ou $R_2$ représente un radical alkyle ou alcényle ramifié.

5. Composés selon l'une des revendications 1 à 4, dans lesquels les radicaux $R_1$ et $R_2$ ont la même signification.

6. Composés selon l'une des revendications précédentes, dans lesquels chacun des radicaux $R_3$ et $R_4$ est un radical alkyle ayant jusqu'à 4 atomes de carbone.

7. Composés selon l'une des revendications précédentes, dans lesquels chacun des radicaux $R_3$ et/ou $R_4$ est un radical alkyle non-ramifié.

8. Composés selon l'une des revendications précédentes, dans lesquels $R_3$ et $R_4$ ont la même signification.

9. Composés selon l'une des revendications 1 à 5, dans lesquels $R_3$ et $R_4$ forment en commun un enchaînement alkylène $-(CH_2)_n-$, avec $n = 3$ à 5.

10. Médicament contenant au moins un composé selon la revendication 1 ou ses sels d'addition avec un acide, pharmaceutiquement utilisables.

11. Procédé de préparation de composés de formule générale I, dans laquelle $R_1$, $R_2$, $R_3$ et $R_4$ ont les significations données dans la revendication 1, et de leurs sels d'addition avec un acide, pharmaceutiquement utilisable, caractérisé en ce que

a) on réduit des composés substitués analoguement de formule générale

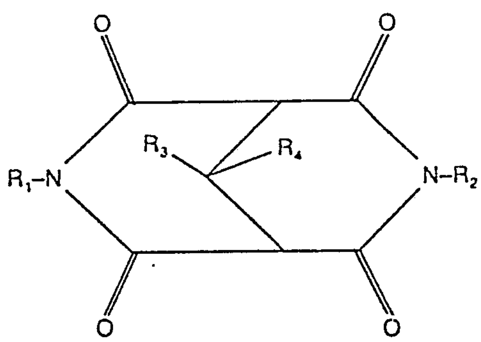 (II)

dans laquelle $R_1$, $R_2$, $R_3$ et $R_4$ ont les significations ci-dessus, ou

b) on fait réagir des composés de formule générale IIIa

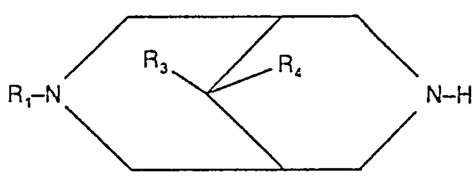 (IIIa)

dans laquelle $R_1$, $R_3$ et $R_4$ ont les significations ci-dessus, avec des composés de formule $R_2X$ dans laquelle $R_2$ a la signification ci-dessus et X représente un groupe éliminable connu en soi, ou

c) pour préparer des composés de formule générale I dans laquelle $R_1$ et $R_2$ sont identiques, on fait réagir des composés de formule générale IIIb

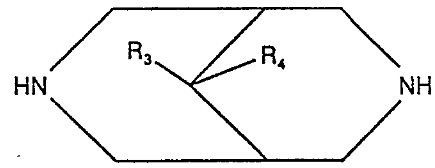 (IIIb)

dans laquelle $R_3$ et $R_4$ ont les significations ci-dessus, avec des composés de formule générale $R_1X$ dans laquelle $R_1$ et X ont les significations ci-dessus,

et que, si on le souhaite, on convertit les composés ainsi obtenu de formule générale I en leurs sels d'addition avec un acide, parmaceutiquement utilisables.

**Revendications pour l'état contractant AT**

1. Procédé de préparation de composés de formule générale I

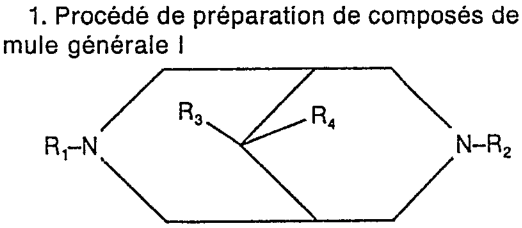 (I)

dans laquelle

$R_1$ et $R_2$, indépendamment l'un de l'autre, sont chacun un radical alkyle ayant de 1 à 12 atomes de carbone, alcényle ayant 2 à 12 atomes de carbone ou cycloalkyle ayant de 3 à 6 atomes de carbone, le radical cycloalkyle étant lié à l'atome N directement ou par l'intermédiaire d'un radical alkylène ayant de 1 à 3 atomes de carbone, de préférence 1 atome de carbone, et

$R_3$ et $R_4$, indépendamment l'un de l'autre, sont chacun un radical alkyle ayant de 1 à 7 atomes de carbone, ou forment ensemble un enchaînement alkylène $-(CH_2)_n-$, avec $n = 3$ à 6,

à la condition que les radicaux $R_1$ à $R_4$ contiennent au total au moins 5 atomes de carbone,

et leurs sels d'addition avec un acide, pharmaceutiquement utilisables, caractérisé en ce que

a) on réduit des composés substitués analoguement de formule générale

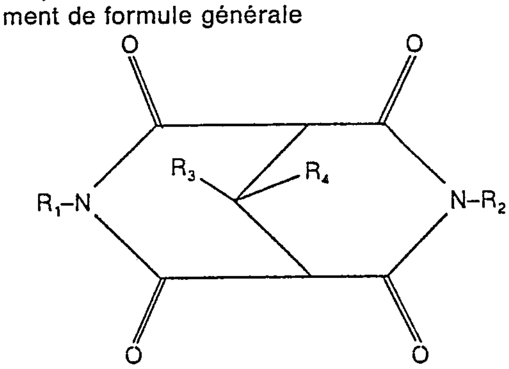 (II)

dans laquelle $R_1$, $R_2$, $R_3$ et $R_4$ ont les significations ci-dessus, ou

b) on fait réagir des composés de formule générale IIIa

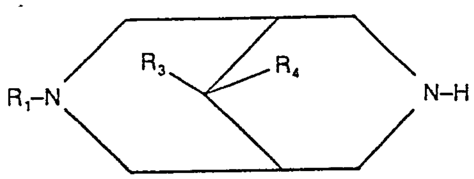

(IIIa)

dans laquelle $R_1$, $R_3$ et $R_4$ ont les significations ci-dessus, avec des composés de formule $R_2X$ dans laquelle $R_2$ a la signification ci-dessus et X représente un groupe éliminable connu en soi, ou

c) pour préparer des composés de formule générale I dans laquelle $R_1$ et $R_2$ sont identiques, on fait réagir des composés de formule générale IIIb

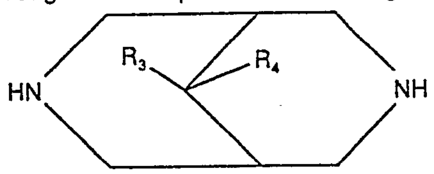

(IIIb)

dans laquelle $R_3$ et $R_4$ ont les significations ci-dessus, avec des composés de formule générale $R_1X$ dans laquelle $R_1$ et X ont les significations ci-dessus,

et que, si on le souhaite, on convertit les composés ainsi obtenus de formule générale I en leurs sels d'addition avec un acide, pharmaceutiquement utilisables.

2. Procédé selon la revendication 1, caractérisé en ce qu'on prépare des composés de formule générale I dans lesquels chacun des radicaux $R_1$ et/ou $R_2$ contient un groupe alkyle ou cycloalkylalkyle ayant jusqu'à 7 atomes de carbone, ou un groupe cycloalkyle ayant jusqu'à 6 atomes de carbone.

3. Procédé selon l'une des revendications 1 ou 2, caractérisé en ce qu'on prépare des composés de formule générale I dans lesquels chacun des radicaux $R_1$ et/ou $R_2$ représente un radical alkyle ou alcényle non-ramifié.

4. Procédé selon l'une des revendications 1 ou 2, caractérisé en ce qu'on prépare des composés de formule générale I dans lesquels chacun des radicaux $R_1$ et/ou $R_2$ représente un radical alkyle ou alcényle ramifié.

5. Procédé selon l'une des revendications 1 à 4, caractérisé en ce qu'on prépare des composés de formule générale I dans lesquels les radicaux $R_1$ et $R_2$ ont la même signification.

6. Procédé selon l'une des revendications précédentes, caractérisé en ce qu'on prépare des composés de formule générale I, dans lesquels chacun des radicaux $R_3$ et $R_4$ est un radical alkyle ayant jusqu'à 4 atomes de carbone.

7. Procédé selon l'une des revendications précédentes, caractérisé en ce qu'on prépare des composés de formule générale I dans lesquels chacun des radicaux $R_3$ et/ou $R_4$ est un radical alkyle non-ramifié.

8. Procédé selon l'une des revendications précédentes, caractérisé en ce qu'on prépare des composés de formule générale I dans lesquels $R_3$ et $R_4$ ont la même signification.

9. Procédé selon l'une des revendications 1 à 5, caractérisé en ce qu'on prépare des composés de formule générale I dans lesquels $R_3$ et $R_4$ forment en commun un enchaînement alkylène $-(CH_2)_n-$, avec $n = 3$ à 5.